# EUROPÄISCHE PATENTSCHRIFT

(11) **EP 0 683 146 B1**
(45) Veröffentlichungstag und Bekanntmachung des Hinweises auf die Patenterteilung: **10.09.1997**
(21) Anmeldenummer: 95106748.7
(22) Anmeldetag: 04.05.1995
(51) Int. Cl.: C07C 7/04, C07C 7/11, C10G 70/04

(54) **Verfahren zur Trennung von C2/C3-Kohlenwasserstoffen in Ethylenanlagen**
Process for the separation of C2/C3 hydrocarbons in ethylene production plants
Procédé de séparation d'hydrocarbures en C2/C3 dans des unités de production d'éthylène

(30) Priorität: 19.05.1994 DE 4417584
(43) Veröffentlichungstag der Anmeldung: 22.11.1995
(73) Patentinhaber: Linde Aktiengesellschaft, 65189 Wiesbaden (DE)
(72) Erfinder: Pham Duc, Tuat, Dipl.-Ing., D-82377 Penzberg (DE); Walzl, Roland, Dipl.-Ing., D-82340 Feldafing (DE)
(74) Vertreter: Kasseckert, Rainer

(56) Entgegenhaltungen:
- US-A- 3 783 126
- HYDROCARBON PROCESSING, August 1979 HOUSTON US, Seiten 101-107, K. D. MIKULLA 'Front-end deethanizer saves energy'

## Beschreibung

Die Erfindung betrifft ein Verfahren zur Trennung von C₂₋ /C₃₊ -Kohlenwasserstoffen in Ethylenanlagen, wobei das die C₂₋ /C₃₊ -Kohlenwasserstoffe enthaltende Rohgas verdichtet wird, wobei aus der der Rohgasverdichtung nachgeschalteten Vorkühlung ein gasförmiger Strom und zumindest ein Kondensatstrom anfallen und wobei mittels zweier bei unterschiedlichem Druck betriebenen Kolonnen die C₂₋ -Kohlenwasserstoffe und die C₃₊ -Kohlenwasserstoffe getrennt gewonnen werden.

Olefinanlagen, wegen ihres Hauptproduktes auch Ethylenanlagen genannt, sind das Herzstück eines jeden Petrochemiekomplexes. Sie liefern die petrochemischen Zwischenprodukte, die in weiterverarbeitenden Anlagen zu Chemieprodukten (Polyethylen, Polypropylen, ABS-Kunststoffe, Polystyrol etc.) verarbeitet werden.

Der Ethylenprozeß besitzt eine zentrale Stellung in der Kette der Gewinnung von Chemieprodukten und ist deshalb von besonderer Bedeutung. Die Ethylenanlage ist eine Anlage zur Gewinnung von olefinischen Kohlenwasserstoffen. Paraffinische oder naphthenische Kohlenwasserstoffschnitte, die aus Erdgas oder Erdöl abgetrennt wurden, werden thermisch gespalten. Aus dem dabei entstehenden Rohgas werden im Zerlegungsteil der Anlage die gewünschten Produkte abgetrennt und gereinigt.

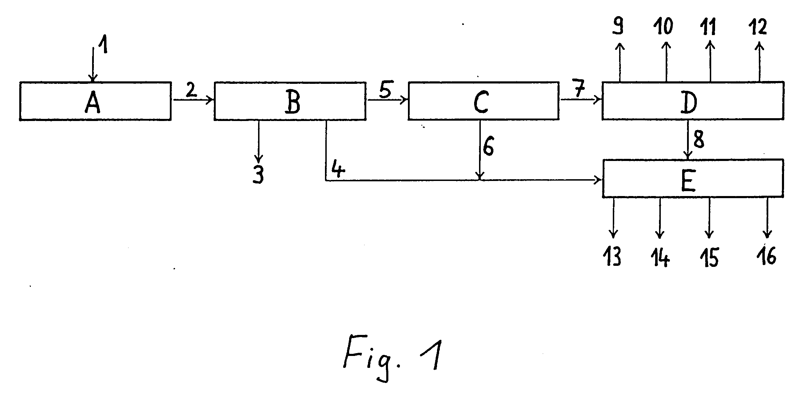

- In Figur 1:: wird ein vereinfachtes Blockschaltbild des Ethylenprozesses dargestellt.

Wie aus diesem Blockschaltbild von Figur 1 zu entnehmen ist, läßt sich der Ethylenprozeß in 5 Einheiten A bis E gliedern:
A: die Spaltung,
B: die Ölfraktionierung und Wasserwäsche,
C: die Rohgasverdichtung,
D: die Rohgastrennung und
E: die C₃₊ -Aufarbeitung.

Der Einsatz 1, bestehend aus gesättigten Kohlenwasserstoffen, wird im Beisein von Wasserdampf thermisch gespalten (A) und anschließend zum Unterbrechen der Reaktionen abgekühlt. Das Produkt aus diesem Schritt ist das Rohgas 2, bestehend im wesentlichen aus einer Mischung aus Wasserstoff, gesättigten und ungesättigten Kohlenwasserstoffen und Wasserdampf.

In der Öl- und Wasserwäsche B wird das Rohgas 2 gekühlt und von Öl- und schweren Benzinkomponenten (Pyrolyseöl 3) gereinigt. Außerdem wird der Großteil des im Spaltgas enthaltenen Wasserdampfes kondensiert und nach Wiederverdampfung zur Spaltung zurückgeführt (nicht gezeigt). Es wird ferner ein schwerere Kohlenwasserstoffe (C₃₊ -Kohlenwasserstoffe) enthaltendes Kondensat 4 abgetrennt und in die C₃₊ -Verarbeitung E gegeben.

Das vorgereinigte Gas 5 wird mehrstufig in der Rohgasverdichtung C auf einen höheren Druck, beispielsweise ca. 36 bar, verdichtet, damit es mittels kryogener Prozeßstufen in seine Einzelprodukte zerlegt werden kann. Es wird dabei ein C₃₊ - Kohlenwasserstoffe enthaltendes Kondensat 6 abgetrennt und mit dem Kondensat 4 in die C₃₊ -Verarbeitung E gegeben.

In der Rohgastrennung D wird in mehreren kryogenen Trennschritten das Rohgas 7 in die C₃₊ -Fraktion 8, sowie in die Einzelprodukte Ethylen 12, Ethan 11, Methan 10 und Wasserstoff 9 zerlegt. Die Trennung erfolgt durch partielle Kondensation des Rohgases, sowie durch drei Fraktionierungen, von denen eine die nachfolgend im Detail behandelte C₂ - / C₃ -Trennung, auch Deethanizer genannt, ist. Ein wichtiger Bestandteil dieser Einheit ist auch die C₂-Hydrierung, in der Acetylen durch eine katalytische Hydrierung in Ethylen 12 und Ethan 11 umgesetzt wird.

Die C₃₊ -Aufarbeitung beinhaltet die Verarbeitung der C₃ - und schwereren Kohlenwasserstoffe, die in die Produkte Propylen 13, Propan 14, C₄ -Kohlenwasserstoffe 15 und Pyrolysebenzin 16 zerlegt werden.

Die Prinzipien der Prozeßführung sind für die Anlagenblöcke A bis C und E bei den bekannten Ethylenprozessen weitgehend identisch. Sie unterscheiden sich in der Regel nur in Ausführungsdetails.

Ein sehr wichtiger Prozeßschritt liegt im Rohgastrennteil D der Ethylenanlage, insbesondere in der C₂₋/C₃₊ -Trennung in diesem Prozeß. Es sind verschiedene Möglichkeiten der Anordnungen innerhalb dieses Prozeßschrittes entwickelt worden.

Im Bereich der Rohgastrennung D, die aus den drei Trennschritten C₁ / C₂ - Trennung (Demethanizer), C₂ / C₃ -Trennung (Deethanizer), C₂H₄ / C₂H₆ -Trennung, sowie aus der C₂ -Hydrierung und der Wasserstoffreinigung besteht, bieten sich verschiedene Möglichkeiten an, diese Prozeßschritte untereinander zu kombinieren. Die unterschiedliche Anordnung der Trennschritte und der Hydrierung prägt auch den Charakter der Anlage. Dies schlägt sich auch in den Bezeichnungen nieder. So wird beispielsweise vom "Front-end-Deethanizer"- oder vom "Front-end-Demethanizer"-Verfahren gesprochen.

Die Wahl der Anordnung der Trennschritte hängt im wesentlichen von drei Faktoren ab.

Erstens wird versucht, die Investitionskosten zu minimieren. Die Rohgastrennung zählt auf Grund der vorherrschenden Prozeßparameter (tiefe Temperaturen bis ca. -160°C und Drücke bis ca. 40 bar) zu den kapitalintensivsten Baugruppen der Anlage. Die genannten Prozeßparameter erfordern den Einsatz von teueren Werkstoffen und Apparaten, wobei das Verfahren inclusive der Kältekreisläufe ca.50 bis 60 Apparate erforderlich machen kann.

Zweitens soll der Energieverbrauch reduziert werden und so Einsparungen erzielt werden. Die Trennung des Rohgases erfordert hohe Energieumsätze bei tiefen Temperaturen und die Bereitstellung von großen Kältemengen auf verschiedenen Temperaturniveaus. Für die Spaltgaszerlegung einer 300.000-Jahrestonnen-Anlage sind für Rohgasverdichtung und Kältekreisläufe beispielsweise mehr als 25 MW Kompressorleistung erforderlich.

Drittens gilt es, die Betriebsweise zu vereinfachen. Ein übergeordneter Aspekt, der bei allen Optimierungsbestrebungen beachtet werden muß, ist dabei die Sicherheit und Betreibbarkeit der Anlage. Es gilt, zu starke Prozeßverschachtelungen zu vermeiden, die den Betrieb der Anlage beeinträchtigen und sie störungsanfälliger machen. Diesbezüglich ist vor allem das Resultat der Optimierung der Wärmeintegration kritisch zu betrachten. Es gilt auch, die Wahl der Prozeßparameter so zu treffen, daß die Bildung von unerwünschten Nebenprodukten unterbleibt (Polymerablagerungen, Grünöl etc.) und die Laufzeit der Anlage nicht beeinträchtigt wird.

Eine Optimierung wird allerdings dadurch erschwert, daß die Investitionskosten und der Energieverbrauch gegenläufige Tendenzen aufweisen. Denn zusätzliche Energieeinsparung erfordert den Einsatz von mehr Wärmeaustauschfläche oder von mehr Trennschritten und führt damit zwangsläufig zu höheren Investitionskosten.

Im Bereich der Rohgaszerlegung konnten sich im wesentlichen zwei Prozesse durchsetzen, die sich durch die Anordnung des Deethanizers und der C₂ -Hydrierung im Prozeßablauf voneinander unterscheiden: Das Front-end-Demethanizer-Verfahren und das Front-end-Deethanizer-Verfahren. Das Front-end-Deethanizer-Verfahren weist jedoch gegenüber dem Front-end-Demethanizer-Verfahren verschiedene Vorteile auf:
- Die Investitionskosten sind geringer, da insbesondere in Verbindung mit dem offenen Ethylenkreis die Zahl der Apparate und die Summe der Heizflächen kleiner ist.
- Der Energieverbrauch der drei Großverdichter, die die Trennleistung für die Gaszerlegung liefern, ist ca 5 % kleiner.
- Alle schweren Bestandteile des Rohgases werden vor Eintritt in den Tieftemperaturteil abgeschieden, so daß dieser Anlagenteil und die Methankolonne nicht mit C₃ - und schwereren Komponenten belastet wird. Aus sicherheitstechnischer Sicht bedeutet dies, daß die Bildung von explosiven Harzen, die durch Reaktionen von NOₓ, mit Butadien oder C₅ -Dienen im Tieftemperaturteil entstehen können, weitgehend ausgeschlossen werden kann.
- Die Acetylenhydrierung, die bei diesem Verfahren im gesamten C₂₋ -Strom durchgeführt wird, weist Katalysatorlaufzeiten von mehr als fünf Jahren auf, gegenüber Laufzeiten von 6 Monaten bis zu einem Jahr bei der Hydrierung im reinen C₂ -Strom. Außerdem ist die Bildung von Grünöl stark reduziert. Die Hydrierung erfordert keine Zugabe von gereinigtem Wasserstoff, so daß der Prozeß schneller angefahren werden kann. Es entfällt die Nachreinigung des C₂ - Stromes von Methan und Wasserstoff, so daß ein Prozeßschritt eingespart wird.

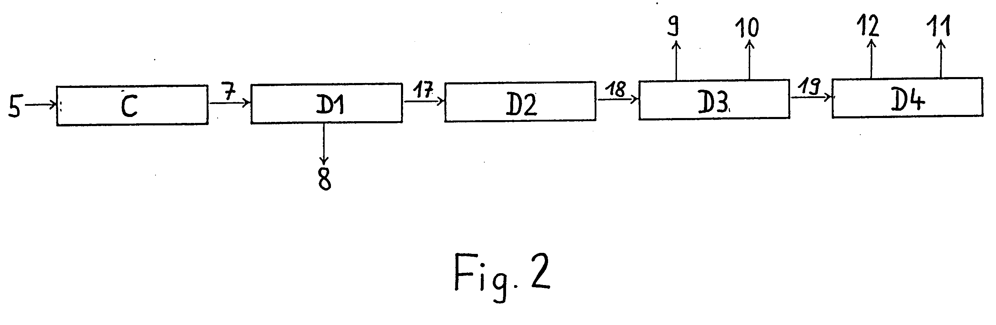

Im folgenden wird anhand von
- Figur 2:: ein Schema des bevorzugten und vorteilhaften Front-end-Deethanizer-Verfahrens beschrieben.

Das Rohgas 5 wird zunächst verdichtet (C). Nach der Verdichtung C wird das Rohgas 7 in der Vorkühlung (in Stufe D1 enthalten) auf ca. -30 bis -40°C gekühlt. Ausfallende Kondensate 8 und nicht kondensiertes Gas werden in einem ersten Trennschritt D1, dem Deethanizer, in zwei Fraktionen zerlegt, wobei die eine Fraktion 17 die C₂ - Kohlenwasserstoffe und leichteren Komponenten enthält und die andere Fraktion 8 aus den C₃ -Kohlenwasserstoffen und schwereren Komponenten besteht. Der C₂ - Kohlenwasserstoffstrom 17 wird einem Hydrierschritt D2 zugeführt, bei dem das Acetylen mit Teilen des im Gas enthaltenen Wasserstoffes zu Ethylen und Ethan reagiert. Der acetylenfreie Strom 18 wird in der Tiefkühlung D3 weiter gekühlt und partiell kondensiert, bis die erforderliche Wasserstoffreinheit des Wasserstoffs in Leitung 9 erreicht ist. Die anfallenden Kondensate werden einer Methankolonne (ebenfalls in Stufe D3) zugeführt, in der Methan 10 von C₂ -Kohlenwasserstoffen 19 getrennt wird. Das Sumpfprodukt dieser Kolonne besteht aus der sauberen C₂ -Kohlenwasserstofffraktion 19, die in dem nachgeschalteten Niederdruck-C₂ -Splitter in Stufe D4 in den Ethylenproduktstrom 12 und den Ethanstrom 11 getrennt wird. Da die C₂ -Kohlenwasserstofffraktion zur C₂ -Trennkolonne (Splitter) nicht mit Methan oder Wasserstoff verunreinigt ist, kann der C₂ -Splitter über eine Wärmepumpenschaltung betrieben werden, deren Verdichter mit einer Verdichterstufe des Ethylen-Kältekreises kombiniert ist.

Der Schlüsselschritt des beschriebenen Verfahrens ist der "Front-end-Deethanizer", der den Einsatz der "Front-end-Hydrierung" und des offenen Ethylenkreislaufes ermöglicht.

Die zentrale Rolle dieses Prozeßschrittes sowie seine Bedeutung für den Energieverbrauch und den Betrieb der Anlage machte den Deethanizer im Laufe der Entwicklungen des Verfahrens vielfach zum Gegenstand von Untersuchungen und Verbesserungen, in deren Folge sich das Erscheinungsbild dieses Prozeßschrittes mehrfach änderte. Die in Figur 2 gezeigte Stufe D1 der Vorkühlung und insbesondere der Trennung von C₂₋ / C₃₊ -Kohlenwasserstoffen ist daher bezüglich der Verbesserung des Verfahrens von besonderer Bedeutung.

In der Veröffentlichung "Front-end deethanizer saves energy", K.D. Mikulla, Hydrocarbon Processing, August 1979, Seiten 101 bis 107, wird das Front-end-Deethanizer-Verfahren beschrieben. Zunächst wurde der Deethanizer als Einzelkolonne gebaut (siehe beispielsweise Bild 4 der Veröffentlichung). Die Einspeisungen in die Kolonne bestehen aus den Kondensaten der Vorkühlung, die auf die Kolonne gepumpt werden, sowie aus dem nicht kondensierten Rohgas. Die Kolonne arbeitet bei vollem Rohgasdruck. Sie wird im Sumpf mit Dampf beheizt und im Kopf gekühlt. Verbesserungen des Einkolonnensystems führten zur Entwicklung des Zweikolonnensystems, das sich in vielen Ethylenanlagen gut bewährt hat. Als wesentliche Verbesserung wird die Trennung der C₂ - und C₃ -Komponenten in zwei, in ihrem Arbeitsdruck abgestuften Kolonnen durchgeführt (siehe beispielsweise Bild 3 der Veröffentlichung). Die Einspeisung der Kondensate und des nicht kondensierten Rohgases erfolgt in eine erste Kolonne (Absorber-Stripper-Kolonne), die bei vollem Rohgasdruck arbeitet. Die Kolonne erfüllt die Doppelfunktion, zur Trennung der C₂₋ -und C₃₊ -Komponenten Methan und leichteren Komponenten (C₂ -Kohlenwasserstoffe) auszustrippen und C₃ -Kohlenwasserstoffe und schwerere Komponenten aus dem Gasstrom zu absorbieren. Als Strippgas wird ein in einem Aufkocher erwärmter Gasstrom eingesetzt. Ein Teil der im Rohgas enthaltenen C₂ -Komponenten geht mit in das Sumpfprodukt. Das methanarme Sumpfprodukt der ersten Kolonne wird in eine zweite Kolonne eingespeist, dem eigentlichen Deethanizer. Dort wird bei niedererem Druck der Strom aus der ersten Kolonne in einen C₂ - und einen C₃₊ -Strom zerlegt. Das Kopfprodukt dieser zweiten Kolonne, bestehend aus den im Einspeisestrom enthaltenen C₂ -Komponenten, wird nach Unterkühlung teilweise als Rücklauf zur Absorption in die erste Kolonne gegeben. Es wird ein ständiger C₂ -Kreislauf über beide Kolonnen aufrechterhalten, der in der ersten Kolonne die aufsteigenden C₃₊ - Komponenten absorbiert und in der zweiten Kolonne wieder vom den C₃₊ -Kohlenwasserstoffen getrennt wird. Dadurch, daß das Kopfgas der zweiten Kolonne weitgehend frei von Methan ist, kann die Kondensation des Rücklaufes bei höheren Temperaturen erfolgen, was gegenüber der Einfachkolonne zu einer deutlichen Energieeinsparung führt. Durch den tieferen Kolonnendruck in der zweiten Kolonne stellt sich gegenüber der Einfachkolonne eine Sumpftemperatur ein, bei der die Polymerbildung der ungesättigten Komponenten stark reduziert ist und eine längere Laufzeit der Kolonne ohne Reinigung erreicht werden kann.

Sind die Vorteile des Zweikolonnensystems gegenüber der Einfachkolonne auch unbestritten, so bleibt doch die Tatsache bestehen, daß die Investitionskosten dabei höher liegen als beim Einkolonnenverfahren. Insbesondere bei Neuanlagen ist der Anlagenbau im internationalen Wettbewerb einem extremen Kostendruck ausgesetzt, was dazu führt, daß Maßnahmen, die zwar zu einer Verbesserung des Anlagenbetriebes führen, aber andererseits die Investitionskosten erhöhen, schwer durchzusetzen sind.

Der Erfindung liegt daher die Aufgabe zugrunde, ein Verfahren der eingangs genannten Art aufzuzeigen, welches einerseits ermöglicht, die apparativen Kosten der Ethylenanlage gering zu halten, andererseits aber einen verhältnismäßig geringen Energieverbrauch sicherstellt und dabei eine möglichst vereinfachte Betriebsweise erlaubt.

Insbesondere sollen dabei die Vorteile der beiden bekannten Verfahren (Ein-und Zweikolonnenverfahren) hinsichtlich der Investitionskosten, des Energieverbrauchs und der Betriebsfreundlichkeit erhalten bleiben bzw. möglichst vereinigt und verbessert werden. Das Verfahren soll aber eine effektive Trennung der C₂₋ / C₃₊ -Kohlenwasserstoffe gewährleisten.

Diese Aufgabe wird erfindungsgemäß dadurch gelöst, daß der gasförmige Strom aus der Vorkühlung in die bei höherem Druck betriebene Kolonne und jeder Kondensatstrom aus der Vorkühlung in die bei niedererem Druck betriebene Kolonne eingespeist werden.

Die wesentlichen Vorteile dieser erfindungsgemäßen Verfahrensführung liegen in den reduzierten Investitionskosten bei maximal gleichem Energieverbrauch verglichen mit dem bekannten Zweikolonnensystem. Es entfallen die kalten Pumpenpaare in der Vorkühlung, da die Kondensate mit Eigendruck in die zweite Kolonne, die bei niedererem Druck arbeitet, eingespeist werden. Die Sumpftemperatur der bei niedererem Druck betriebenen Kolonne (Niederdruckkolonne) entspricht dabei im wesentlichen der des bekannten Zweikolonnensystems.

Besondere Vorteile können mit dem erfindungsgemäßen Verfahren dadurch erzielt werden, daß die bei höherem Druck betriebene Kolonne (Hochdruckkolonne) ausschließlich als Absorptionskolonne zur Absorption der C₃₊ -Kohlenwasserstoffe betrieben wird. Zu Nachteilen im Verfahren führt die Einspeisung der Kondensate in die Niederdruckkolonne und die dadurch ermöglichte Beschränkung der Funktion der Hochdruckkolonne auf die Absorption der C₃₊ -Kohlenwasserstoffe überraschenderweise nicht. Zwar werden auch teilweise C₂₋ -Kohlenwasserstoffe in der Hochdruckkolonne absorbiert. Allerdings kann die Hochdruck- oder Absorptionskolonne wesentlich kleiner gebaut werden, was sich in merklichen Kosteneinsparungen im apparativen Aufwand der Anlage niederschlägt. Durch die erfindungsgemäße Verfahrensführung kann die Bauhöhe der Hochdruckkolonne auf in der Regel sogar mehr als ein Drittel der Bauhöhe der Absorptions- und Strippkolonne nach dem bislang bekannten Zweikolonnenverfahren der C₂₋ -/ C₃₊ -Trennung reduziert werden. Die C₃₊ -Kohlenwasserstoffe können in der Hochdruckkolonne problemlos absorbiert werden, so daß eine C₂₋ -Kohlenwasserstofffraktion frei von C₃₊ - Komponenten vom Kopf der bei höherem Druck betriebenen Kolonne abgezogen werden kann.

Das erfindungsgemäße Verfahren verfolgt weiter die Idee des Zweikolonnensystems bei unterschiedlichen Arbeitsdrücken, jedoch mit dem Unterschied, daß der Hochdruckteil nur eine Absorberfunktion für die C₃₊ -Komponenten hat. Die Kondensate aus der Vorkühlung werden zusammen mit dem Sumpfprodukt der Hochdruckkolonne direkt in die Niederdruckkolonne eingespeist. Der nicht kondensierte Rohgasstrom wird zum Sumpf der Absorberkolonne (Hochdruckkolonne) geführt. Der Rücklauf der Niederdruckkolonne zur Hochdruckkolonne wird wie im bekannten Verfahren aus einem Teilstrom des Kopfproduktes der Niederdruckkolonne gewonnen. Dieser Rücklaufstrom absorbiert in der Hochdruckkolonne die noch im Rohgas enthaltenen C₃₊ -Komponenten und läuft anschließend als Sumpfprodukt von der Hochdruckkolonne in die Niederdruckkolonne ab.

Ein Ausstrippen der C₂₋ -Komponenten in der Hochdruckkolonne (Absorptionskolonne) kann entfallen. Im Sumpfproduktstrom von der Hochdruckkolonne in die Niederdruckkolonne enthaltene C₂₋ -Kohlenwasserstoffe werden in der Niederdruckkolonne aus dem Flüssigstrom befreit und über den Kopfstrom aus der Niederdruckkolonne abgezogen. Bevorzugt weist deshalb die bei höherem Druck betriebene Kolonne keine Sumpfheizung auf und wird insbesondere nicht über einen Aufkocher beheizt, was zu einer Senkung der erforderlichen Investitions- und Betriebskosten beiträgt. Vorteilhafterweise wird in der bei höherem Druck betriebenen Kolonne (Hochdruckkolonne) eine Temperaturdifferenz zwischen Kopf- und Sumpftemperatur der Kolonnne von 1 bis 20 K, vorzugsweise zwischen 3 und 10 K, eingestellt.

In Weiterbildung der Erfindung wird der Kopfstrom der Niederdruckkolonne abgekühlt und zumindest teilweise kondensiert. Die Kühlung des Kopfstroms der Niederdruckkolonne kann mittels eines Plattenwärmetauschers durchgeführt werden. Dabei kann der Kopfstrom der Niederdruckkolonne gegen Propylen- und/oder Ethylenkälte, aber auch gegen die verdampfende C₂₋ -Kohlenwasserstofffraktion vom Kopf der Hochdruckkolonne im indirekten Wärmetausch gekühlt werden. Diese Maßnahme bedeutet zusätzliche Energieeinsparungen.

Die Einspeiseströme in die Niederdruckkolonne sind außer den im wesentlichen enthaltenen C₃₊ -Komponenten mit C₂₋ -Kohlenwasserstoffen, insbesondere mit Methan, beladen. Bei der Kondensation des Kopfstromes der Niederdruckkolonne werden diese leichten Kohlenwasserstoffe nicht auskondensiert und es resultiert ein kleiner gasförmiger Reststrom. Dieser Reststrom wird vorteilhafterweise in die Rohgasverdichtung zurückgeführt, und zwar bevorzugt nach Anwärmung in der Vorkühlungsstufe. Der methanreiche Reststrom enthält auch Inertgase und umfaßt einen Volumenstrom von etwa wenigen hundert kg/h.

Die Niederdruckkolonne wird mit einer Sumpfheizung, bevorzugt einem Aufkocher, ausgestattet. Dadurch wird in der Niederdruckkolonne bzw. der Deethanizer-Kolonne Ausstrippen der C₂₋ -Komponenten sichergestellt. Damit ergibt sich ein vom Sumpf der Niederdruckkolonne abgezogener Flüssigstrom aus im wesentlichen C₃₊ -Kohlenwasserstoffen, der bis auf Verunreinigungen in der Größenordnung von wenigen ppm frei von C₂₋ -Kohlenwasserstoffen ist.

Als vorteilhaft haben sich die nachfolgend angegebenen Betriebsbedingungen in den beiden Kolonnen erwiesen: In der Hochdruckkolonne sollte bei einem Druck zwischen 25 und 50 bar absolut, vorzugsweise zwischen 30 und 40 bar absolut, und bei einer Kopftemperatur zwischen -60 und -30°C, vorzugsweise zwischen - 50 und -40°C, und bei einer Sumpftemperatur zwischen -40 und -20°C, vorzugsweise zwischen -40 und -30°C, gearbeitet werden. Die Hochdruckkolonne enthält vorteilhafterweise zwischen 5 und 25 Kolonnenböden, vorzugsweise zwischen 10 und 15 Kolonnenböden. In der Niederdruckkolonne wird mit Vorteil ein Druck zwischen 15 und 40 bar absolut, vorzugsweise zwischen 20 und 30 bar absolut, eine Temperaturdifferenz zwischen Kopf- und Sumpftemperatur der Niederdruckkolonne von 90 bis 120 K, vorzugsweise zwischen 100 und 120 K, und eine Kopftemperatur zwischen -40 und -10°C, vorzugsweise zwischen - 30 und -20°C, und eine Sumpftemperatur zwischen +70 und +110°C, vorzugsweise zwischen +80 und +90°C, eingestellt. In der bei niedererem Druck betriebenen Kolonne sind vorteilhafterweise zwischen 50 und 75 Kolonnenböden, vorzugsweise zwischen 60 und 70 Kolonnenböden, eingebaut.

Das erfindungsgemäße Verfahren ist für die Verarbeitung von Rohgasen einsetzbar, die sowohl von der Spaltung von flüssigen- als auch gasförmigen Einsätzen stammen.

Ein weiterer betrieblicher Vorteil gegenüber dem bekannten Zweikolonnenverfahren besteht in dem einfacheren Regelverhalten des Systems. Die Regelung des bekannten Zweikolonnensystems erfordert insbesondere beim Anfahren viel Fingerspitzengefühl vom Operator, da das einzuhaltende Temperaturfenster im Sumpf der Hochdruckkolonne zwischen der Gefahr eines Methandurchbruchs auf der einen Seite und andererseits der Möglichkeit einer Verarmung des internen C₂ -Kreises relativ schmal ist. Bei dem erfindungsgemäßen Verfahren mit einem Hochdruckabsorber stört das Methan in der Niederdruckkolonne nicht, so daß ein stabiles Regelverhalten gegeben ist.

Die nachfolgende Tabelle 1 gibt einige charakterisierende Daten für das erfindungsgemäße Verfahren wieder. Die angegeben Größen beziehen sich auf eine Ausführungsbeispiel einer Ethylenanlage für eine Naphthaspaltung mit einer Jahreskapazität von 300.000 t Ethylen.

**Tabelle 1**

| | |
|---|---|
| Durchmesser der Niederdruckkolonne in m | 2,8/2,05 |
| Durchmesser der Hochdruckkolonne in m | 1,75 |
| Höhe der Niederdruckkolonne in m | 43 |
| Höhe der Hochdruckkolonne in m | 9 |
| Bodenzahlen der Niederdruckkolonne | 64 |
| Bodenzahlen der Hochdruckkolonne | 13 |
| Kopftemperatur der Niederdruckkolonne | -24°C |
| Sumpftemperatur der Niederdruckkolonne | +86°C |
| Kopftemperatur der der Hochdruckkolonne | -43°C |
| Sumpftemperatur der Hochdruckkolonne | -36°C |
| Druck der Niederdruckkolonne in bar absolut | 26 |
| Druck der Hochdruckkolonne in bar absolut | 36 |
| Gesamtverdichterleistung in kW | 24.970 |

Die Erfindung wird im folgenden anhand des bereits mit Tabelle 1 angegebenen Ausführungsbeispieles näher erläutert.

Hierbei zeigt:

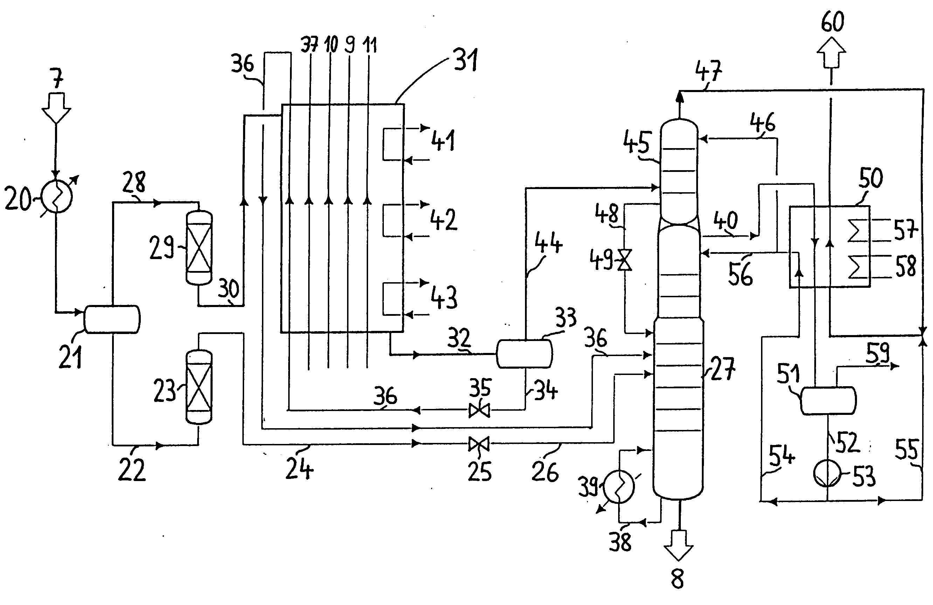

- Figur 3:: ein erfindungsgemäßes Zweikolonnensystem (Deethanizer mit C₃₊ - Absorber) mit Vorkühlung.

Das in Figur 3 dargestellte Verfahrensschema zeigt die Verfahrensstufe D1 aus Figur 2 zum Ausführungsbeispiel nach Tabelle 1.

Das Rohgas 7 vom Verdichter (siehe Verdichtungsstufe C aus Figur 2) wird im Kühler 20 beispielsweise mit Propylen auf eine Temperatur von +12°C abgekühlt und in den Abscheider 21 gegeben. Das Kondensat wird vom Abscheider 21 über Leitung 22 in den Rohgaskondensattrockner 23 geleitet, wo das Kondensat mittels eines Molekularsiebs getrocknet wird. Vom Kondensattrockner 23 wird das Kondensat über Leitung 24 nach seiner Entspannung 25 auf niedereren Druck via Leitung 26 in die Niederdruckkolonne 27 eingespeist.

Der gasförmige Strom vom Abscheider 21 wird über Leitung 28 in den mit einem Molekularsieb ausgestatteten Rohgastrockner 29 gegeben. Über Leitung 30 wird der getrocknete Gasstrom in die Vorkühlung 31 geleitet. Der gekühlte Gasstrom wird über Leitung 32 in den Abscheider 33 eingespeist. Der Kondensatstrom 34 vom Abscheider 33 wird entspannt (35), über Leitung 36 durch den Vorkühler 31 geführt, wobei er durch Teilverdampfung entstehende Kälte abgibt. Anschließend wird der Kondensatstrom 36 in die Niederdruckkolonne 27 gegeben. Im Vorkühler 31 wird beispielsweise die Kälte des Inertgasstromes 37, des Methanstroms 10, des Wasserstoffstroms 9 und/oder des Ethanstroms 11 ausgenützt. Zusätzlich wird beispielsweise mit drei Propylenströmen 41, 42 und 43 gekühlt, wobei der Propylenstrom 41 eine Temperatur von 0°C, der Prophylenstrom 42 eine Temperatur von -20°C und der Prophylenstrom 43 eine Temperatur von -35°C aufweisen.

Der gekühlte Gasstrom 44 vom Abscheider 43 wird in die Absorptionskolonne 45 geleitet. Über Leitung 46 wird ein Kohlenwasserstoffstrom auf den Kopf der Hochdruckkolonne 45 gegeben, um die C₃₊ -Kohlenwasserstoffe auszuwaschen. Vom Kopf der Hochdruckkolonne 45 wird über Leitung 47 ein im wesentlichen aus C₂₋ -Kohlenwasserstoffen bestehender Gasstrom mit einer Temperatur von -43°C abgezogen. Vom Sumpf der Hochdruckkolonne 45 wird der Flüssigstrom 48 mit einer Temperatur von etwa -36°C entspannt (49) und in die Niederdruckkolonne 27 eingespeist.

Im Sumpfbereich der Niederdruckkolonne 27 ist ein Aufkocher 38 vorhanden, der beispielsweise mit Niederdruckdampf (39) beheizt wird, so daß sich eine Sumpftemperatur in der Niederdruckkolonne von +86°C einstellt. Vom Sumpf der Niederdruckkolonne 27 wird über Leitung 8 ein C₃₊ -Kohlenwasserstoffe enthaltender Strom abgezogen und in eine C₃ / C₄ -Trennung gegeben wird, wie sie beispielsweise von der in Figur 1 in Einheit E dargestellten C₃₊ -Aufarbeitung umfaßt wird.

Der Gasstrom vom Kopf der Niederdruckkolonne 27 mit einer Temperatur von -24°C in Leitung 40 wird im Plattenwärmetauscher 50 gekühlt und in den Abscheider 51 geleitet. Das dabei anfallende Kondensat in Leitung 52 wird verdichtet (53) und in die Teilströme 54 und 55 aufgeteilt. Teilstrom 54 wird über den Plattenwärmetauscher 50 geführt und zum einen Teil über Leitung 56 zum Kopf der Niederdrucksäule 27 geleitet, zum anderen Teil über Leitung 46 in die Hochdruckkolonne 45 gegeben. Im Plattenwärmetauscher 50 ist ein indirekter Wärmetausch gegen einen Propylenstrom 57 mit einer Temperatur von -35°C und gegen einen Ethylenstrom 58 mit einer Temperatur von -58°C vorgesehen. Die vom Abscheider 51 abgezogene Gasfraktion 59 kann beispielsweise über Leitung 37 über den Vorkühler 31 zum Rohgasverdichter (vgl. Rohgasverdichter C in den Figuren 1 und 2) zurückgeführt werden. Bevorzugt wird die Gasfraktion 59 in die letzte Verdichterstufe (beispielsweise die fünfte) des Rohgasverdichters eingespeist.

Der zweite Teilstrom des Kondensats in Leitung 55 wird mit dem Kopfstrom der Hochdruckkolonne 45 in Leitung 47 vermischt und über Leitung 60 in eine nicht dargestellte C₂ -Hydrierung geführt.

Die Hochdruckkolonne 45 kann, wie in Figur 3 gezeigt, auf der Niederdruckkolonne 27 aufgesetzt oder aber getrennt von dieser aufgestellt sein.

## Patentansprüche

1. Verfahren zur Trennung von C₂₋ / C₃₊ -Kohlenwasserstoffen in Ethylenanlagen, wobei das die C₂₋ / C₃₊ -Kohlenwasserstoffe enthaltende Rohgas (5) verdichtet wird, wobei aus der der Rohgasverdichtung (C) nachgeschalteten Vorkühlung (31) ein gasförmiger Strom (44) und zumindest ein Kondensatstrom (26, 36) anfallen und wobei mittels zweier bei unterschiedlichem Druck betriebenen Kolonnen (27, 45) die C₂₋ -Kohlenwasserstoffe (60) und die C₃₊ -Kohlenwasserstoffe (8) getrennt gewonnen werden, **dadurch gekennzeichnet,** daß der gasförmige Strom (44) aus der Vorkühlung (31) in die bei höherem Druck betriebene Kolonne (45) und jeder Kondensatstrom (26, 36) aus der Vorkühlung (31) in die bei niedererem Druck betriebene Kolonne (27) eingespeist werden.

2. Verfahren nach Anspruch 1, dadurch gekennzeichnet, daß die bei höherem Druck betriebene Kolonne (45) ausschließlich als Absorptionskolonne zur Absorption der C₃₊ -Kohlenwasserstoffe betrieben wird.

3. Verfahren nach einem der Ansprüche 1 oder 2, dadurch gekennzeichnet, daß die bei höherem Druck betriebene Kolonne (45) keine Sumpfheizung aufweist und insbesondere nicht über einen Aufkocher beheizt wird.

4. Verfahren nach einem der Ansprüche 1 bis 3, dadurch gekennzeichnet, daß der Kopfstrom (40) der bei niedererem Druck betriebenen Kolonne (27) abgekühlt (50) und zumindest teilweise kondensiert (51) wird.

5. Verfahren nach Anspruch 4, dadurch gekennzeichnet, daß der Kopfstrom (40) der bei niedererem Druck betriebenen Kolonne (27) mittels eines Plattenwärmetauschers (50) abgekühlt wird.

6. Verfahren nach einem der Ansprüche 4 oder 5, dadurch gekennzeichnet, daß der Kopfstrom (40) der bei niedererem Druck betriebenen Kolonne (27) gegen Propylen, Ethylen und/oder verdampfende C₂₋ -Kohlenwasserstoffe (60) gekühlt wird.

7. Verfahren nach einem der Ansprüche 4 bis 6, dadurch gekennzeichnet, daß ein nach der Kondensation des Kopfstromes (50, 51) der Niederdruckkolonne (27) verbleibender gasförmiger Reststrom (59) in die Rohgasverdichtung (C) gegeben wird, bevorzugt nach seiner Anwärmung (37) in der Vorkühlung (31).

8. Verfahren nach einem der Ansprüche 1 bis 7, dadurch gekennzeichnet, daß der Kopfstrom (47) der bei höherem Druck betriebenen Kolonne (45) im wesentlichen C₂₋ -Kohlenwasserstoffe enthält und frei von C₃₊ -Kohlenwasserstoffen ist.

9. Verfahren nach einem der Ansprüche 1 bis 8, dadurch gekennzeichnet, daß vom Sumpf der bei niedererem Druck betriebenen Kolonne (27) ein Flüssigstrom (8) aus im wesentlichen C₃₊ -Kohlenwasserstoffen abgezogen wird, der frei von C₂₋ -Kohlenwasserstoffen ist.

10. Verfahren nach einem der Ansprüche 1 bis 9, dadurch gekennzeichnet, daß in der bei höherem Druck betriebenen Kolonne (45) ein Druck zwischen 25 und 50 bar absolut, vorzugsweise zwischen 30 und 40 bar absolut, eingestellt wird.

11. Verfahren nach einem der Ansprüche 1 bis 10, dadurch gekennzeichnet, daß in der bei höherem Druck betriebenen Kolonne (45) eine Temperaturdifferenz zwischen Kopf- und Sumpftemperatur der Kolonnne (45) von 1 bis 20 K, vorzugsweise zwischen 3 und 10 K, vorliegt.

12. Verfahren nach einem der Ansprüche 1 bis 11, dadurch gekennzeichnet, daß in der bei höherem Druck betriebenen Kolonne (45) eine Kopftemperatur zwischen -60 und -30°C, vorzugsweise zwischen - 50 und -40°C, und eine Sumpftemperatur zwischen -40 und -20°C, vorzugsweise zwischen -40 und -30°C, eingestellt wird.

13. Verfahren nach einem der Ansprüche 1 bis 12, dadurch gekennzeichnet, daß in der bei höherem Druck betriebenen Kolonne (45) zwischen 5 und 25 Kolonnenböden, vorzugsweise zwischen 10 und 15 Kolonnenböden, eingebaut sind.

14. Verfahren nach einem der Ansprüche 1 bis 13, dadurch gekennzeichnet, daß in der bei niedererem Druck betriebenen Kolonne (27) ein Druck zwischen 15 und 40 bar absolut, vorzugsweise zwischen 20 und 30 bar absolut, eingestellt wird.

15. Verfahren nach einem der Ansprüche 1 bis 14, dadurch gekennzeichnet, daß in der bei niedererem Druck betriebenen Kolonne (27) eine Temperaturdifferenz zwischen Kopf- und Sumpftemperatur der Kolonne (27) von 90 bis 120 K, vorzugsweise zwischen 100 und 120 K, vorliegt.

16. Verfahren nach einem der Ansprüche 1 bis 15, dadurch gekennzeichnet, daß in der bei niedererem Druck betriebenen Kolonne (27) eine Kopftemperatur zwischen -40 und -10°C, vorzugsweise zwischen - 30 und -20°C, und eine Sumpftemperatur zwischen +70 und +110°C, vorzugsweise zwischen +80 und +90°C, eingestellt wird.

17. Verfahren nach einem der Ansprüche 1 bis 16, dadurch gekennzeichnet, daß in der bei niedererem Druck betriebenen Kolonne (27) zwischen 50 und 75 Kolonnenböden, vorzugsweise zwischen 60 und 70 Kolonnenböden, eingebaut sind.

## Claims

1. Process for separating C₂₋/C₃₊ hydrocarbons in ethylene plants, with the crude gas (5) containing the C₂₋/C₃₊ hydrocarbons being compressed, with one gaseous stream (44) and at least one condensate stream (26, 36) arising from the precooling (31) downstream of the crude gas compression (C) and with the C₂₋ hydrocarbons (60) and the C₃₊ hydrocarbons (8) being separated by means of two columns (27, 45) operated at different pressures, characterized in that the gaseous stream (44) from the precooling (31) is fed into the column (45) operated at higher pressure and each condensate stream (26, 36) from the precooling (31) is fed into the column (27) operated at lower pressure.

2. Process according to Claim 1, characterized in that the column (45) operated at higher pressure is operated solely as an absorption column for absorbing the C₃₊ hydrocarbons.

3. Process according to one of Claims 1 or 2, characterized in that the column (45) operated at higher pressure does not have bottom heating and, in particular, is not heated via a reboiler.

4. Process according to one of Claims 1 to 3, characterized in that the overhead stream (40) of the column (27) operated at lower pressure is cooled (50) and at least partially condensed (51).

5. Process according to Claim 4, characterized in that the overhead stream (40) of the column (27) operated at lower pressure is cooled by means of a plate heat exchanger (50).

6. Process according to one of Claims 4 or 5, characterized in that the overhead stream (40) of the column (27) operated at lower pressure is cooled against propylene, ethylene and/or evaporating C₂₋ hydrocarbons (60).

7. Process according to one of Claims 4 to 6, characterized in that a gaseous residual stream (59) remaining after the condensation of the overhead stream (50, 51) of the low-pressure column (27) is added to the crude gas compression (C), preferably after the residual stream is heated (37) in the precooling (31).

8. Process according to one of Claims 1 to 7, characterized in that the overhead stream (47) of the column (45) operated at higher pressure comprises essentially C₂₋ hydrocarbons and is free of C₃₊ hydrocarbons.

9. Process according to one of Claims 1 to 8, characterized in that a liquid stream (8) of essentially C₃₊ hydrocarbons is taken off from the bottom of the column (27) operated at lower pressure, which liquid stream (8) is free of C₂₋ hydrocarbons.

10. Process according to one of Claims 1 to 9, characterized in that, in the column (45) operated at higher pressure, a pressure between 25 and 50 bar absolute, preferably between 30 and 40 bar absolute, is set.

11. Process according to one of Claims 1 to 10, characterized in that, in the column (45) operated at higher pressure, there is a temperature difference between top temperature and bottom temperature of the column (45) of 1 to 20 K, preferably between 3 and 10 K.

12. Process according to one of Claims 1 to 11, characterized in that, in the column (45) operated at higher pressure, a top temperature is set between -60 and -30°C, preferably between -50 and -40°C, and a bottom temperature is set between -40 and -20°C, preferably between -40 and -30°C.

13. Process according to one of Claims 1 to 12, characterized in that between 5 and 25 column trays, preferably between 10 and 15 column trays, are built into the column (45) operated at higher pressure.

14. Process according to one of Claims 1 to 13, characterized in that, in the column (27) operated at lower pressure, a pressure between 15 and 40 bar absolute, preferably between 20 and 30 bar absolute, is set.

15. Process according to one of Claims 1 to 14, characterized in that, in the column (27) operated at lower pressure, there is a temperature difference between top temperature and bottom temperature of the column (27) of 90 to 120 K, preferably between 100 and 120 K.

16. Process according to one of Claims 1 to 15, characterized in that, in the column (27) operated at lower pressure, a top temperature is set between -40 and -10°C, preferably between -30 and -20°C, and a bottom temperature is set between +70 and +110°C, preferably between +80 and +90°C.

17. Process according to one of Claims 1 to 16, characterized in that between 50 and 75 column trays, preferably between 60 and 70 column trays, are built into the column (27) operated at lower pressure.

## Revendications

1. Procédé de séparation d'hydrocarbures en C₂₋/C₃₊ dans des unités d'éthylène, le gaz brut (5) contenant les hydrocarbures en C₂₋/C₃₊ étant comprimé, un courant gazeux (44) et au moins un courant de condensat (26, 36) se formant à partir de la réfrigération préalable (31) intercalée après la compression du gaz brut (C), et les hydrocarbures en C₂₋ (60) et les hydrocarbures en C₃₊ (8) étant obtenus séparément grâce à deux colonnes fonctionnant à une pression différente (27, 45), caractérisé en ce que le courant gazeux (44) en provenance de la réfrigération préalable (31) est injecté dans la colonne fonctionnant à la pression plus élevée (45), et chaque courant de condensation (26, 36) est injecté à partir de la réfrigération préalable (31) dans la colonne fonctionnant à la pression plus basse (27).

2. Procédé selon la revendication 1, caractérisé en ce que l'on fait fonctionner la colonne fonctionnant à la pression plus élevée (45) exclusivement en tant que colonne d'absorption en vue de l'absorption des hydrocarbures en C₃₊.

3. Procédé selon l'une quelconque des revendications 1 ou 2, caractérisé en ce que la colonne fonctionnant à la pression plus élevée (45) ne présente aucun chauffage de fond et n'est en particulier pas chauffée par l'intermédiaire d'un rebouilleur.

4. Procédé selon l'une quelconque des revendications 1 à 3, caractérisé en ce que le courant de tête (40) refroidit (50) la colonne fonctionnant à la pression plus basse (27), et est au moins en partie condensé (51).

5. Procédé selon la revendication 4, caractérisé en ce que le courant de tête (40) de la colonne fonctionnant à la pression plus basse (27) est refroidi par l'intermédiaire d'un échangeur thermique à plaques (50).

6. Procédé selon l'une quelconque des revendications 4 ou 5, caractérisé en ce que le courant de tête (40) de la colonne fonctionnant à la pression plus basse (27) est refroidi contre du propylène, de l'éthylène, et/ou des hydrocarbures en C₂₋ qui s'évaporent.

7. Procédé selon l'une quelconque des revendications 4 à 6, caractérisé en ce que l'on achemine un courant résiduel gazeux (59) restant après la condensation du courant de tête (50, 51) de la colonne basse pression (27) dans la compression du gaz brut (C), de préférence après son réchauffement (37) dans le refroidissement préalable (31).

8. Procédé selon l'une quelconque des revendications 1 à 7, caractérisé en ce que le courant de tête (47) de la colonne fonctionnant à la pression plus élevée (45) contient essentiellement des hydrocarbures en C₂₋ et est exempt d'hydrocarbures en C₃₊.

9. Procédé selon l'une quelconque des revendications 1 à 8, caractérisé en ce que l'on soutire du fond de la colonne fonctionnant à la pression plus basse (27), un courant fluide (8) essentiellement d'hydrocarbures en C₃₊, qui est exempt d'hydrocarbures en C₂₋.

10. Procédé selon l'une quelconque des revendications 1 à 9, caractérisé en ce que l'on règle, dans la colonne fonctionnant à la pression plus élevée (45), une pression absolue comprise entre 25 et 50 bar, de préférence comprise entre 30 et 40 bar.

11. Procédé selon l'une quelconque des revendications 1 à 10, caractérisé en ce qu'il existe, dans la colonne fonctionnant à la pression plus élevée (45), une différence de température entre la température de la tête et la température du fond de la colonne (45) de 1 à 20 K, de préférence comprise entre 3 et 10 K.

12. Procédé selon l'une quelconque des revendications 1 à 11, caractérisé en ce que l'on règle, dans la colonne fonctionnant à la pression plus élevée (45), une température de tête comprise entre -60 et -30°C, de préférence entre -50 et -40°C, et une température de fond comprise entre -40 et -20°C, de préférence comprise entre -40 et - 30°C.

13. Procédé selon l'une quelconque des revendications 1 à 12, caractérisé en ce que l'on incorpore, dans la colonne fonctionnant à la pression plus élevée (45), entre 5 et 25 plateaux de colonne, de préférence entre 10 et 15 plateaux de colonne.

14. Procédé selon l'une quelconque des revendications 1 à 13, caractérisé en ce que l'on règle, dans la colonne fonctionnant à la pression plus basse (27), une pression absolue comprise entre 15 et 40 bar, de préférence comprise entre 20 et 30 bar.

15. Procédé selon l'une quelconque des revendications 1 à 14, caractérisé en ce qu'il existe, dans la colonne fonctionnant à la pression plus basse (27), une différence de température entre la température de la tête et la température du fond de la colonne (27) de 90 à 120 K, de préférence comprise entre 100 et 120 K.

16. Procédé selon l'une quelconque des revendications 1 à 15, caractérisé en ce que l'on règle, dans la colonne fonctionnant à la pression moins élevée (27), une température de tête comprise entre -40 et -10°C, de préférence entre -30 et -20°C, et une température de fond comprise entre +70 et +110°C, de préférence comprise entre +80 et +90°C.

17. Procédé selon l'une quelconque des revendications 1 à 16, caractérisé en ce que l'on incorpore, dans la colonne fonctionnant à la pression plus basse (27), entre 50 et 75 plateaux de colonne, de préférence entre 60 et 70 plateaux de colonne.
